# EUROPEAN PATENT SPECIFICATION

(11) **EP 2 889 305 B1**
(45) Date of publication and mention of the grant of the patent: **08.03.2017**
(21) Application number: 13830582.6
(22) Date of filing: 15.08.2013
(51) Int. Cl.: C07K 14/78, C07K 14/785, C07K 14/435

(54) **METHOD FOR FRACTIONALLY EXTRACTING MUCIN AND COLLAGEN**
VERFAHREN ZUR FRAKTIONIERTEN EXTRAKTION VON MUCIN UND KOLLAGEN
PROCÉDÉ D'EXTRACTION FRACTIONNAIRE DE MUCINE ET DE COLLAGÈNE

(30) Priority: 23.08.2012 JP 2012184241
(43) Date of publication of application: 01.07.2015
(73) Proprietor: Jellyfish Research Laboratories, Inc., Kawasaki-shi, Kanagawa 213-0012 (JP); Maruwayushi Co., Ltd., Tokyo 141-0031 (JP)
(72) Inventor: BABA, Takayuki, Kawasaki-shi Kanagawa 213-0012 (JP)
(74) Representative: Håmsø Patentbyrå ANS
(86) International application number: PCT/JP2013/004866
(87) International publication number: WO 2014/030323

(56) References cited:
- EP-A1- 1 947 111
- WO-A1-2007/020889
- JP-A- 2004 099 513
- JP-A- 2005 330 257
- JP-A- 2007 051 191
- JP-A- 2007 051 191
- JP-A- 2008 031 106
- JP-A- 2011 520 927

## Description

### TECHNICAL FIELD

The present invention relates to a method for fractionally extracting mucin and collagen, more particularly to a method capable of carrying out a fractional extraction of mucin and a fractional extraction of collagen, both at high efficiency, from a jellyfish as a single raw material.

### BACKGROUND ART

As conventional technology, patent document 1 teaches "a method for extracting a useful substance from an aquatic organism comprising a first step of breaking and beating an aquatic organism to pieces to liberate an enzyme from the aquatic organism; a second step of making the enzyme act on a protein contained in the pieces to decompose the pieces to a liquid substance; and a third step of extracting a useful substance from the liquid substance." Aquatic organisms disclosed in patent document 1 include jellyfish (see paragraphs [0027] to [0038] of the document). Examples of the useful substance extracted by the method disclosed in patent document 1 include those such as alkaline proteases and collagens (see paragraph [0020] of patent document 1). However, the examples do not include "mucin".

Patent document 2 discloses a mucin-type glycoprotein having a structure which is made by 3 to 2000-time repetition of a constitutional repeating unit, or a sequence, consisting of particular eight amino acids, wherein a sugar chain composed of at least one monosaccharide is bonded with at least one amino acid residue in the unit (see claim 1 of patent document 2). Patent document 2 teaches that this mucin-type glycoprotein is produced by a method comprising a step of cutting a solid part of a jellyfish; a step of subjecting the cut jellyfish to extraction with a salt solution; a step of separating crude mucin from the extract by centrifugal separation and dialysis; and a step of purifying the crude mucin to produce a mucin-type glycoprotein. A specific method for producing a mucin-type glycoprotein comprises making pieces of a jellyfish that have been prepared by cutting the jellyfish swollen with water; subjecting the swollen pieces to extraction with a salt water having a small concentration by shaking the pieces in the salt water; adding to the extract ethanol in an amount of three times the amount of the extract to produce precipitate in the form of a gel; separating the precipitate from matter containing the precipitate by centrifugal separation; dissolving the precipitate in water to form a supernatant and separating the supernatant; purifying the supernatant by dialysis; and freezing and drying the purified supernatant (see paragraph [0088] of patent document 2). Although patent document 2 discloses the method for producing a mucin-type glycoprotein, it teaches nothing about collagen or methods for producing it.

Patent document 3 discloses "a method for collecting collagen from jellyfish comprising a freezing step of freezing jellyfish, a thawing step of thawing the frozen jellyfish to let the jellyfish begin decomposition of themselves by activating an endogenous enzyme that the jellyfish themselves have, a stirring step of stirring the thawed jellyfish in order to solubilize collagen of the jellyfish in an unmodified state to produce a neutral salt solution containing the collagen in the unmodified state; and a collecting step of collecting the collagen in the unmodified state from the neutral salt solution (see claims of patent document 3). Patent document 3 is silent about separating and collecting mucin from jellyfish.

Patent document 4 discloses "a method for collecting collagen from jellyfish comprising a low-temperature storing step of storing jellyfish at a predetermined low temperature in order to activate an endogenous enzyme that the jellyfish themselves have so as to let the jellyfish begin decomposition of themselves whereby collagen that the jellyfish have is solubilized in an unmodified state and a neutral salt solution containing the unmodified collagen is produced; and a collecting step of collecting the unmodified collagen from the neutral salt solution (see claim 1 of patent document 4). The method for collecting collagen disclosed in patent document 4 is based on the finding that the enzyme to solubilize collagen works when the jellyfish is stored at a low temperature (see paragraph [0019] of patent document 4). It is taught in this document that the low temperature in the low-temperature storing step should be in the range of -2°C to 25°C (see paragraph [0021] of patent document 4).

### PRIOR ART DOCUMENTS

### [Patent Documents]

Patent Document 1: JP 2001-178492 A
Patent Document 2: WO 2007/020889
Patent Document 3: JP 2007-051191 A
Patent Document 4: JP 2008-31106 A

### DISCLOSURE OF THE INVENTION

### [Problems to be Solved by the Invention]

The objective of the present invention is to provide a method for fractionally extracting mucin and collagen from a jellyfish as a single raw material.

### [Means to Solve the Problems]

Means to achieve the objective are as follows:
(1) A method for fractionally extracting mucin and collagen including:
   (A) a protein-separating process comprising breaking a jellyfish to provide broken pieces containing water; subj ecting the broken pieces to first solid-liquid separation to provide a liquid component; mixing the liquid component with a water-soluble alcohol having 1 to 4 carbon atoms to prepare a first mixture wherein the water-soluble alcohol has a concentration of at least 50% by volume to a whole of the first mixture; and subjecting the first mixture to second solid-liquid separation to provide solids, and
   (B) a fractionating process comprising mixing the solids, which have been provided in the protein-separating process (A), with an aqueous solution of a water-soluble alcohol having 1 to 4 carbon atoms wherein a concentration of the alcohol in the aqueous solution is from 10 to 40% by mass, to provide a second mixture; and subj ecting the secondmixture to third solid-liquid separation whereby the second mixture is fractionally divided into a mucin-containing liquid constituent and a collagen-containing solid constituent.
(2) The method for fractionally extracting mucin and collagen according to item (1), the protein-separating process (A) further comprising washing the solids provided by the second solid-liquid separation with a water-soluble alcohol having 1-4 carbon atoms and/or water to provide washed solids, which are supplied to the fractionating process (B).
(3) The method for fractionally extracting mucin and collagen according to item (2), wherein the water-soluble alcohol having 1-4 carbon atoms and/or water is so mixed with the washed solids produced in the protein-separating process (A) that a mass of the water-soluble alcohol having 1-4 carbon atoms and/or water is 1 to 4 times a mass of the washed solids.

### [Advantages of the Invention]

The present invention provides a method for fractionally extracting mucin and collagen from jellyfish. The present invention provides a method for fractionally extracting mucin and collagen, capable of separating mucin from jellyfish with an enhanced extraction amount, compared with the conventional method.

### BRIEF DESCRIPTION OF THE DRAWINGS

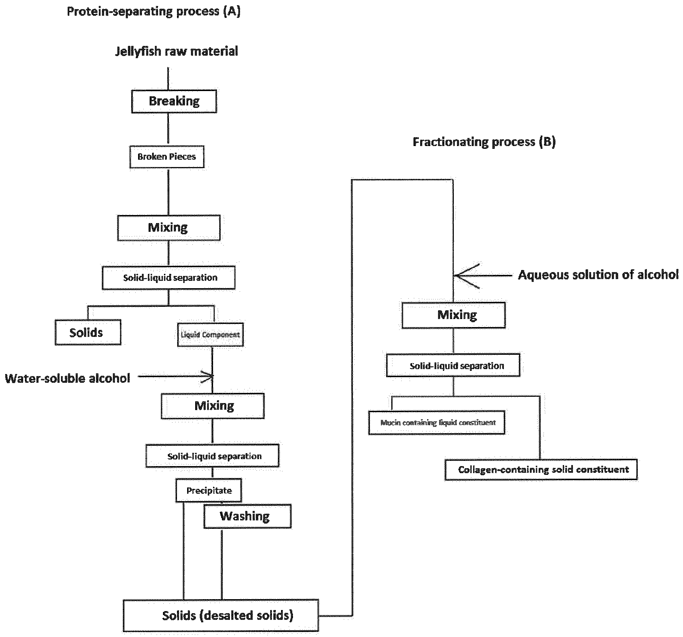

[Figure 1] Figure 1 is a process flow chart showing an example of the fractionally separating method according to the present invention.

### EMBODIMENTS TO CARRY OUT THE INVENTION

The jellyfish used in the present invention is jellyfish belonging to the phylum Cnidaria. Examples of the jellyfish may include water jellyfish, or moon jellyfish (Aurelia aurita), Japanese sea nettles (Chrysaora pacifica), belt jellyfish (Aequorea coerulescens), Nomura's jellyfish (Nemopilema nomurai), box jellyfish (Carybdea rastoni), edible jellyfish (Rhopilema esculenta), and Habu-jellyfish (Chironix yamaguchii). Preferable are jellyfish whose safety to human is ensured. Examples of the preferable jellyfish may include water or moon jellyfish, edible jellyfish, and Nomura's jellyfish, which have been already eaten as food.

There is no special limitation on the state of jellyfish. For example, raw jellyfish, frozen jellyfish, dried jellyfish and salted jellyfish may be used.

Mucin separated from jellyfish by the method according to the present invention has a structural unit which is made by at least 3-time repetition of a constitutional repeating unit that comprises the following amino acid sequence represented by formula (I), or sequence number 1:

Val-Xaa-Glu-Thr-Thr-Ala-Pro (I)

In formula (I), Xaa denotes Val or Ile.

Mucin is a high polymer with a variable molecular weight. The number of repetition is different frommolecular to molecular, even if the mucin is produced by the method of the invention that uses jellyfish belonging to a same species as raw material. The result of a gel filtration analysis, corrected with a number average molecular weight obtained by an amino acid sequence analysis, showed that the molecular weight of a mucin produced by a specific method, which is an example of the method of the invention, was from 10 to 1400 kDa. Studying this result together with the structure of the sugar chain, which will be discussed hereinafter, the inventor supposes that there exists in the natural world mucin of a high polymer with a molecular weight approximately three times as large as the molecular weight of the mucin produced by the present invention that has 3 to 700-time repetition of the constitutional repeating unit. The inventor does not think that mucins with such a large molecular weight have properties and functions that are considerably different from those of the mucins produced by the specific method of the present invention. Therefore mucins for this invention have about 3 to 2000-time repetition, preferably about 3 to 700-time repetition, of the amino acid sequence. Let us suppose that a sugar chain is bonded with all of the threonine (Thr) residues and the sugar chain is -GalNac-Gal, a most typical sugar chain. Then, when the molecular weight of such a mucin is about 4.5 kDa, the number of times of the repetition is about three (3). When the molecular weight of such a mucin is about 750 kDa, the number of times of the repetition is about forty (40). It should be noted that the same supposition will be employed hereinafter when the number of times of the repetition is calculated from a molecular weight, in the absence of an explanatory note to the contrary.

The constitutional repeating units may be bonded with each other directly or through a linker. Although there is no special limitation on the linker, one specific example of it may be an S-S bond formed by cysteine residues.

Mucins produced by the method of the present invention may contain amino acids other than those contained in the amino acid sequence to such a degree that the existence of the other amino acids does not adversely affect functions of mucin, such as viscosity, antibacterial activity, and moisture retention. Furthermore, the sequence of the amino acids forming the constitutional repeating unit in the repeating structure may be shifted. More specifically, the mucin derived from Japanese sea nettles (Chrysaora pacifica) has a constitutional repeating unit of VEXXAAPV where the amino acid sequence of the repeating unit represented by formula (I) is shifted by one amino acid. Therefore mucins separated by the method of the invention may include those which lack one or a few amino acids at the N-terminus of the constitutional repeating unit and therefore whose sequence of the amino acids forming the constitutional repeating unit is shifted. Among these mucins whose sequence of the amino acids forming the constitutional repeating unit is shifted, preferable mucins are those that lack valine (Val) at the N-terminus of the constitutional repeating unit.

The mucin according to the present invention may sometimes have at least one amino acid residue, preferably from one to five amino acid residues, of the constitutional repeating unit which is bonded with a sugar chain composed of at least one monosaccharide, preferably from one to five monosaccharides. Although there is no special limitation on the kind of amino acid residue bonded with the sugar chain, the sugar chain should preferably be bonded with threonine residues (Thr). Mucins separated by the method of this invention may sometimes have amino acid residues bonded with a sugar chain from 98% to 100% of which are threonine (Thr) residues, for example. As explained hereinbefore, because mucin-type glycoproteins are high polymers with a variable molecular weight, the number of amino acid residues with which a sugar chain is bonded varies from molecule to molecule. However, it is supposed that almost all of the threonine residues included in the constitutional repeating units, each of which has two threonine residues as explained hereinbefore, are bonded with a sugar chain. Thus the number of bonded sugar chains in a mucin, separated by the method according to the present invention, varies depending on the number of constitutional repeating units that compose the mucin.

Monosaccharides composing the sugar chain are those found in common mucins. Examples of the monosaccharide may include N-acetylgalactosamine, galactose, N-acetylglucosamine, sialic acid, arabinose, and fucose. In many cases the sugar chain is one composed of N-acetylgalactosamine and/or galactose. Specifically, the threonine residues (Thr) of the constitutional repeating unit are bonded with N-acetylgalactosamine and galactose to form a structural part of Thr-GalNAc-Gal, or they are bonded only with N-acetylgalactosamine to form a structural part of Thr-GalNAc. Such a mucin may be represented, for example, by following formula (II) :

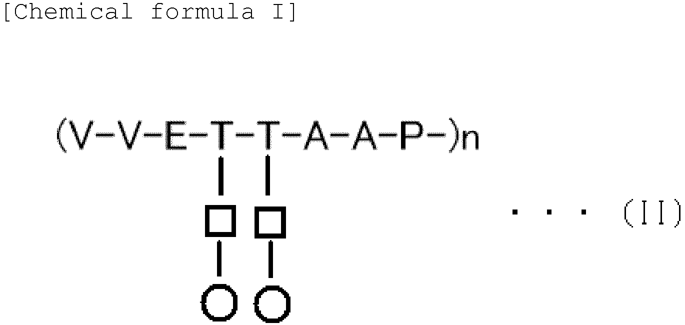

In formula (II), □ denotes GalNAc, and ○ Gal. The monosaccharide Gal, shown by ○, may be missing.

A sugar chain includes normally from 1 to 10 monosaccharides, particularly from 1 to 8 monosaccharides, more particularly from 1 to 5 monosaccharides, bonded therewith in the form of a straight chain or a branched chain. Because the mucin has a variable molecular weight, which is the property of the mucin, the number, kinds, structures, and sizes of sugar chains that a mucin has are different from mucin molecule to mucin molecule. Also, monosaccharides included in one mucin may be different from each other. For example, when a mucin separated from a water jellyfish is compared with another mucin separated from another water jellyfish, the repeating parts of the peptide of the former are identical with those of the peptide of the latter. However, they are different from each other only in the kinds of sugars composing sugar chains and the compositions thereof. Although the sugar chains of the former are different from those of the latter, mucins with same repeating parts of the peptide are considered to function in the same way for jellyfish including water jellyfish (Aurelia aurita) and Japanese sea nettles (Chrysaora pacifica) in the natural world. It is considered that the functions of mucin in water jellyfish are not largely different from those of mucin in Japanese sea nettles. Thus, it is supposed that the sugar chains do not alter main properties of the mucin, but they serve to make fine adjustments to specific properties thereof. Therefore mucins with same repeating parts of the peptide, even though they have different sugar chains, are within the scope of the mucin separated by the method of this invention.

The collagen separated by the method of this invention has such a composition of amino acids that glycine amounts to approximately one-third of the entire amino acids. Therefore a measurement of the amount of glycine contained in a substance separated by the method of this invention will be able to identify whether the substance is a collagen. The collagen separated by the method of the invention, as well as conventionally known collagens, contains hydroxyproline and hydroxylysine as specific amino acids. The collagen extracted from jellyfish has a structure similar to type V-like collagen of mammals and the collagen is formed fromheterotrimetric α1, α2, and α3 chains. One of the characteristics peculiar to the collagen extracted from jellyfish is that it has a larger amount of sugar than other collagens have and it contains more hydroxylysine than hydroxyproline.

The method for fractionally extracting mucin and collagen according to the present invention includes:
(A) a protein-separating process, which may be simply called process (A) hereinafter, comprising breaking a jellyfish to provide broken pieces containing water; subjecting the broken pieces to first solid-liquid separation to provide a liquid component; mixing the liquid component with a water-soluble alcohol having 1 to 4 carbon atoms to prepare a first mixture wherein the water-soluble alcohol has a concentration of at least 50% by volume to a whole of the first mixture; and subj ecting the first mixture to second solid-liquid separation to provide solids, and
(B) a fractionating process, which may be simply called process (B) hereinafter, comprising mixing the solids, which have been provided in the protein-separating process (A), with an aqueous solution of a water-soluble alcohol having 1 to 4 carbon atoms wherein a concentration of the alcohol in the aqueous solution is from 10 to 40% by mass, to provide a second mixture; and subjecting the second mixture to third solid-liquid separation whereby the second mixture is fractionally divided into a mucin-containing constituent liquid and a collagen-containing solid constituent.

In process (A) of the present invention, jellyfish are first broken into broken pieces containing water. Jellyfish to be broken may those caught from the sea, or frozen jellyfish that have been frozen after they were caught from the sea. When frozen jellyfish are used, they should preferably be thawed before they are used for the method of the present invention.

Jellyfish in any state as raw material should be washed with water. Then, the washed jellyfish is divided into a solid and liquid with any means. Preferable examples of the means of the dividing may include centrifugal separators, strainers, drainers, and drying machines.

The solid, the jellyfish, is broken with breaking means, such as scissors, homogenizers, blenders, or shear shredders, into broken pieces containing water. The water-containing broken pieces may be aggregate of particulates formed by the breaking, aggregate of fragments each in the shape of a thread or a strip, aggregate of unshaped fragments with dimensions each approximately from 5 mm to 1 cm, or any possible aggregate.

Process (A) includes stirring the water-containing broken pieces and subjecting the stirred to solid-liquid separation to provide a liquid component. Although the water-containing broken pieces may be allowed to stand, it is preferably stirred. When it is permitted to stand, it should be left normally for 1 to 24 hours. The water-containing broken pieces, or a preparatory mixture of the broken pieces and water, when it is allowed to stand or it is stirred, should preferably have a temperature that should be kept from 0 to 25°C. When the preparatory mixture is left, preferably stirred, water-soluble components of the jellyfish are dissolved in the liquid. Examples of the means to carry out the first solid-liquid separation of the mixture may include centrifugal separators, filters, and squeezers.

Process (A) includes mixing the liquid component, which has been provided through the first solid-liquid separation, with a water-soluble alcohol with 1 to 4 carbon atoms to prepare a first mixture wherein the water-soluble alcohol has a concentration of at least 50% by volume, preferably from 60% by volume to 80% by volume, to a whole of the first mixture.

Examples of the water-soluble alcohol with 1 to 4 carbon atoms may be monovalent alcohols with 1 to 4 carbon atoms, specifically, alkyl alcohols such as methyl alcohol, ethyl alcohol, propyl alcohols, and butyl alcohols. Among them, ethyl alcohol and propyl alcohols are preferable. From the viewpoint of toxicity to the human body, ethanol and propyl alcohols are preferable to methanol.

When the mixing ratio of the liquid component to the water-soluble alcohol is such that the concentration of the water-soluble alcohol to the first mixture of the liquid component and the water-soluble alcohol is less than 50% by volume, mucin does not move into the solids and remains in the liquid component, which results in a failure to achieve the objective of the present invention.

More specific description based on experimental facts is as follows:
When the liquid component and the water-soluble alcohol are mixed to provide such a mixture that the concentration of the water-soluble alcohol to the mixture is 20% by volume, this mixing produces precipitate whose main component is collagen. However, the precipitated collagen is not all of the collagen contained in the liquid component; the liquidportion, separated from the precipitate by solid-liquid separation, which may be called first liquid portion hereinafter, contains the rest of the collagen and mucin. Therefore the mixing of the liquid component and the water-soluble alcohol to prepare a mixture wherein the concentration of the water-soluble alcohol to the mixture is, for example, 20% by volume, which is followed by solid-liquid separation, is not capable of separating collagen from mucin efficiently.
When the first liquid portion and a water-soluble alcohol are mixed to provide such a mixture that the concentration of the water-soluble alcohol to the mixture is 30% by volume, this mixing produces precipitate whose main component is collagen, which is similar to the mixing of the liquid component and the water-soluble alcohol described hereinbefore. However, the precipitated collagen is not all of the collagen contained in the first liquid portion; the liquid portion, separated from the precipitate by further solid-liquid separation, which may be called second liquid portion hereinafter, contains the rest of the collagen and mucin. Therefore the mixing of the first liquidportion and the water-soluble alcohol to prepare a mixture wherein the concentration of the water-soluble alcohol to the mixture is 30% by volume, which is followed by the further solid-liquid separation, is not capable of separating collagen from mucin efficiently.
When the second liquidportion and a water-soluble alcohol are mixed to provide such a mixture that the concentration of the water-soluble alcohol to the mixture is 40% by volume, this mixing produces precipitate that contains several kinds of peptides. It is understood from this result that the second liquid portion contains little collagen. However, the liquid portion, separated from the precipitate by still further solid-liquid separation, which may be called third liquid portion hereinafter, contains mucin. Therefore the mixing of the second liquid portion and the water-soluble alcohol to prepare a mixture wherein the concentration of the water-soluble alcohol to the mixture is 40% by volume, which is followed by the still further solid-liquid separation, is not capable of providing collagen and mucin as precipitates. Thus this way of mixing is not capable of separating collagen and mucin efficiently, either.

When the third liquid portion and a water-soluble alcohol are mixed to provide such a mixture that the concentration of the water-soluble alcohol to the mixture is 50% by volume, this mixing produces precipitate that contains mucin. This result indicates that the third liquid portion contains little collagen.

It is understood from these experimental facts that when the liquid component, obtained by the first solid-liquid separation of the water-containing broken pieces of jellyfish, and the water-soluble alcohol are mixed to prepare such a mixture that the concentration of the water-soluble alcohol to the mixture is 50% by volume, collagen and mucin precipitate as solids, a collection of which provides an efficient separation of collagen and mucin.

These experimental facts were found for the first time by the inventor of the present invention who used a water-soluble alcohol, such as ethanol and propanol, for the extraction. The phenomena observed when ethanol and propanol were employed are also observed when a lower alcohol having 1 to 4 carbon atoms, which is homologous with and near relative to ethanol and propanol, is employed.

This invention is based on these experimental facts.

Some people might think: When the liquid component obtained by first solid-liquid separation of the water-containing broken pieces of jellyfish is mixed with the water-soluble alcohol so that a mixture in such a state that the concentration of the water-soluble alcohol to the mixture is less than 50% by volume is prepared, and the mixture is divided into precipitate to be formed and a filtrate, the precipitate will contain collagen and the filtrate will contain mucin. Then, an addition of the water-soluble alcohol to the filtrate so that the concentration of the water-soluble alcohol to the resulting mixture is not less than 50% by volume will precipitate mucin out of the mixture. However, this method requires complicated operations, because the extraction of collagen from the filtrate with the water-soluble alcohol has to be repeated so that all of the contained collagen is completely extracted. Furthermore, this method is not efficient, because the precipitate contains collagen and peptides and a step of removing peptides from the precipitate is necessary.

The mixing of the liquid component with the water-soluble alcohol should preferably be carried out at a temperature normally in the range of 0 to 25°C. The mixing at a temperature within the range is preferable because it does not affect mucin or collagen adversely. The time period of the mixing is normally from 1 to 48 hours, preferably from 12 to 24 hours.

In process (A), after the mixing of the liquid component with the alcohol the resulting mixture is subjectedto the second solid-liquid separation so that solids are produced. Examples of the means for the second solid-liquid separation may include centrifugal separators, filters, and squeezers.

Solids formed by the second solid-liquid separation may be supplied to next process (B). However, because the solids contain impurities such as salts and lipid, the solids should be washed in order that the impurities are removed and that the concentration of the alcohol contained in the solids is made constant. The process of the washing may sometimes be called washing process (A1).

The washing process (A1) may include a step of mixing the solids provided by the second solid-liquid separation in the protein-separating process (A) with an aqueous solution of a water-soluble alcohol having 1 to 4 carbon atoms in such an amount ratio, from the economical viewpoint, that the mass of the alcohol is one to four times as large as that of the solids.

Examples of the water-soluble alcohol having 1 to 4 carbon atoms maybe monovalent alkyl alcohols with 1 to 4 carbon atoms, specifically, alkyl alcohols such as methyl alcohol, ethyl alcohol, propyl alcohols, and butyl alcohols. Among them, ethyl alcohol and propyl alcohols are preferable.

Although the mixture of the solids and the water-soluble alcohol may be allowed to stand, it should preferably be stirred so that the solids are washed effectively. The time period for the washing, or the time for which the mixture is allowed to stand or for which the mixture is stirred, is normally within one hour. The temperature for the washing, or the temperature at which the mixture is allowed to stand or it is stirred, should preferably from 0 to 25°C. The washing at a temperature within the range is preferable because it does not affect mucin or collagen adversely.

After the mixture is left or stirred, the mixture is subjected to solid-liquid separation. Examples of the means to carry out the solid-liquid separation may include centrifugal separators, filters, and squeezers.

In this washing process (A1) the step of the mixing of the solids with a specified concentration of the water-soluble alcohol and the following step of the solid-liquid separation may be carried once, or may be repeated several times. The number of times of repetition of the washing operation may be decided depending on a change in the mass of the solids. Specifically, the washing operation should be repeated until the change in the mass of the solids is less than 5%; when the change is less than 5%, the washing operation may be stopped and the process may advance to the next step.

The solids after being washed in the washing process (A1) contain a salt in a smaller amount than the solids before being washed. Thus the solids that have been washed in the washing process (A1) may be called washed solids in order to be literally distinguished from solids that are not washed.

In the method of the invention, the washed solids prepared in process (A1), or the solids that have not been subjected to process (A1), are supplied to process (B).

Process (B) comprises mixing the solids produced in the protein-separating process (A), which may have been washed or have not been subjected to the washing process (A1), with an aqueous solution of a water-soluble alcohol having 1 to 4 carbon atoms whose alcohol content is from 10 to 40% by mass to produce a second mixture; and subjecting the second mixture to third solid-liquid separation to divide the second mixture into a mucin-containing liquid constituent and a collagen-containing solid constituent.

Examples of the water-soluble alcohol may be monovalent alkyl alcohols such as methyl alcohol, ethyl alcohol, propyl alcohols, and butyl alcohols. Among them, ethyl alcohol and propyl alcohols are preferable.

When the alcohol content of the aqueous solution of the water-soluble alcohol is from 10% by mass to 40% by mass, the solids separated by the third solid-liquid separation contain collagen and the separated liquid contain mucin. Also, when an aqueous solution of the water-soluble alcohol whose alcohol content is more than 40% by mass is added to the solids and mixed therewith, solids produced by the adding and mixing contain very little amount of peptides other than collagen, which means that the solids contain collagen at high purity.

The mixing of the solids produced in process (A) with the aqueous solution of the water-soluble alcohol may normally be carried out by stirring the mixture at a temperature from 0 to 25°C within one hour. The mixing at a temperature within the specified range is preferable because it does not affect mucin or collagen adversely.

After the completion of the mixing, the mixture is subjected to the third solid-liquid separation that divides the mixture into a mucin-containing liquid constituent that contains mucin and a collagen-containing solid constituent that contains collagen.

Mucin may be isolated from the mucin-containing liquid constituent by known purification such as ion-exchange chromatography and drying such as freeze-drying. What is contained in the mucin-containing liquid constituent or is isolated therefrom may be identified as mucin, when the amino acid analysis reveals that it has the amino acids represented by formula (I), or sequence number 1.

Collagen may be isolated from the collagen-containing solid constituent by known purification such as ion-exchange chromatography and drying such as freeze-drying. The isolated may be identified as collagen by measuring the amount of contained Gly with the amino acid analysis.

### EXAMPLES

The invention will be described by way of working examples that exemplify the invention but do not limit it, which may aid deeper understanding of the invention.

### (Working Example 1)

Mucin and collagen were taken from a water jellyfish (Aurelia aurita) by the following procedure.

1) Wash a jellyfish in a refrigerated state with water, and separate it into a solid and liquid with a strainer.
2) Break the solid separated from the liquid with a shear shredder to prepare broken pieces as sample.
3) Charge an extracting and stirring machine with the sample produced in step 2) and water, and carry out stirring and extraction at 4°C.
4) Subject the aqueous solution, produced in step 3) and kept at 4°C, to centrifugal separation at a centrifugal force of 10000 G for 10 minutes to produce a liquid component.
5) Add isopropyl alcohol to the liquid component produced in step 4) to prepare such a mixture that the concentration of the isopropyl alcohol to the mixture is 70% by volume, to form precipitate in the form of a gel.
6) Stir overnight the liquid containing the gel precipitate kept at 4°C, and subject the stirred to centrifugal separation at a centrifugal force of 10000 G for 10 minutes to produce solids.
7) Add to the solids produced in step 6) a 60% by mass aqueous solution of isopropyl alcohol in such an amount that the mass of the alcohol is three times as large as that of the solids.
8) Stir for 5 minutes at 4°C the mixture formed in step 7), and subject the stirred to centrifugal separation at 4°C at a centrifugal force of 10000 G for 10 minutes to produce solids.
9) Subject the solids that have been treated in step 8) again to the operations of steps 7) and 8).

The protein-separating process (A) was completed, as explained in the preceding paragraph. The next process was: 10) Add to the solids obtained in step 9) a 25% by mass aqueous solution of isopropyl alcohol in such an amount that the mass of the alcohol is three times as large as that of the solids. 11) Stir the mixture produced in step 10) for 5 minutes at 4°C, and subj ect the stirredmixture, being kept at 4°C, to centrifugal separation at a centrifugal force of 10000 G for 10 minutes to produce solids.
12) Subject the solids produced in step 11) to a series of the operations of steps 10) and 11) twice. Divide the treated into a solid constituent and a liquid constituent.
13) Subject the liquid constituent separated in step 12) to dialysis and freeze-drying to produce a final solid product (L) . Place the solid constituent separated in step 12) in water to form a suspension, and subject the suspension to dialysis and freeze-drying to produce a final solid product (S).

An amino acid analysis of the solid product (L) and the solid product (S) was carried out with an automatic amino acid analyzer in accordance with the following procedure to examine the constituent amino acids of the solid product (L) and those of the solid product (S). The analysis revealed that the solid product (L) was mucin and the solid product (S) was collagen.

Each of the solid product (L) and the solid product (S) was purified by the ion-exchange chromatography, which has been described hereinbefore, and further dialyzed. Each sample of the treated products (each in an amount of approximately 12 micrograms) was placed in a hydrolysis tube. Each sample in the hydrolysis tube was dried and hardened with a centrifugal evaporator. Each hydrolysis tube with the sample was placed in an outer tube with a 5.7N solution of constant boiling-point hydrochloric acid in the outer tube. The pressure inside the outer tube was decreased and the tube was sealed. The hydrolysis was carried out by a gas phase method at 110°C for 20 hours.

The outer tube, which had been sealed, was opened. The contents of the hydrolysis tube were dried in the same way. The dried hydrolysate was dissolved in 100 microliter of a 0. 02 N aqueous solution of hydrochloric acid. The amino acid analysis of the hydrolysate was carried out with a High Speed Amino Acid Analyzer L-8500A manufactured by Hitachi Corporation. Amino acids contained in the hydrolysate were divided by using five buffers with an ion-exchange column in accordance with a method of analyzing special amino acids, the method provided by Hitachi Corporation. The divided amino acids were allowed to react with ninhydrin by the post-column method, and the resulting derivatives were analyzed with two visible radiations having different wavelengths. The amount of the amino sugars and ordinary amino acids were determined from peaks on a chromatogram obtained with a visible radiation of 570 nm, and the amount of proline was determined from a corresponding peak on a chromatogram obtained with a visible radiation of 440 nm, based on the data obtained by analyzing 2 moles of a mixture of the standard amino acids, glucosamine, and galactosamine.

The result of the analysis of composition of the amino acids is shown in Table 1. As understood from the column whose header is "Concentration ratio of amino acids", the analysis revealed that the peptide of the produced mucin had such a composition that the ratio of threonine (Thr): glutamic acid (Glu): proline (Pro): alanine (Ala): valine (Val)+isoleucine (Ile): N-acetylgalactosamine (GalNAc) was 2:1:1:2:2:2 each with an error margin of 15%. The analysis ensures that the resultant was mucin of high purity.

Also, the analysis showed that the peptide of the produced collagen had such a composition that the ratio of the concentration of glycine (Gly) to that of the entire amino acids was about 1/3. Thus the analysis confirmed that the produced was collagen of high purity.

The respective yields of the produced mucin and collagen are shown in Table 2.

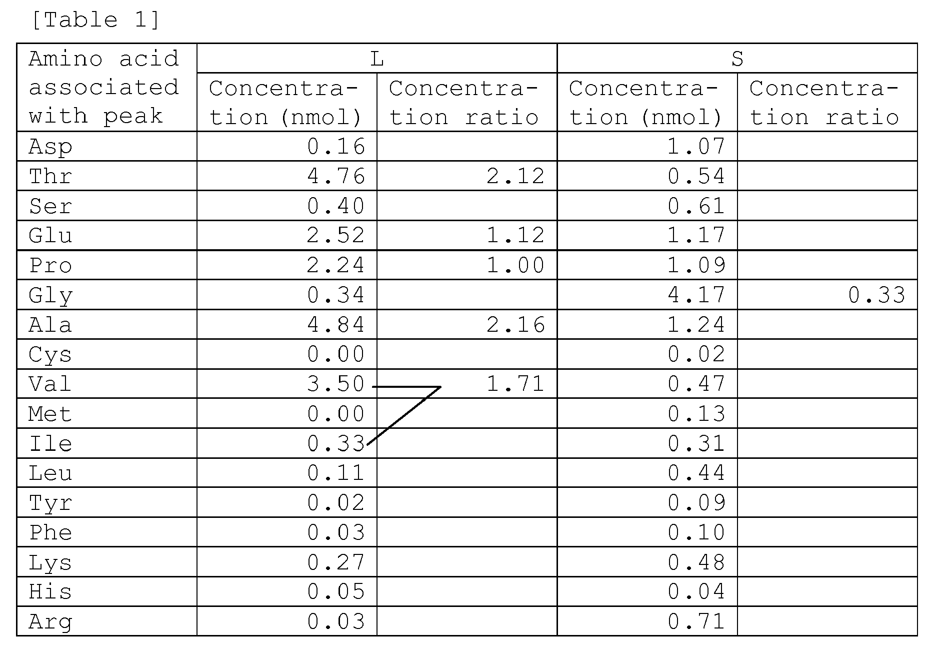

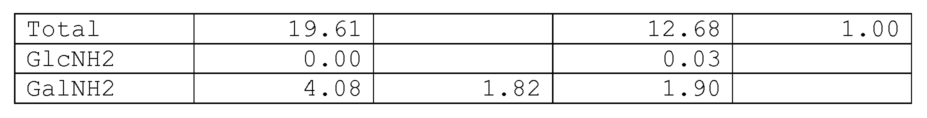

### (Working Example 2)

Mucin and collagen were taken from a water jellyfish (Aurelia aurita) by the following procedure.

1) Wash a jellyfish in a refrigerated state with water, and separate it into a solid and liquid with a strainer.
2) Break the solid separated from the liquid with a shear shredder to prepare broken pieces as sample.
3) Charge an extracting and stirring machine with the sample produced in step 2) and water, and carry out stirring and extraction at 4°C.
4) Subject the aqueous solution, produced in step 3) and kept at 4°C, to centrifugal separation at a centrifugal force of 10000 G for 10 minutes to produce a liquid component.
5) Add ethanol to the liquid component produced in step 4) to prepare such a mixture that the concentration of the ethanol to the mixture is 70% by volume, to form precipitate in the form of a gel.
6) Stir overnight the liquid containing the gel precipitate kept at 4°C, and subject the stirred to centrifugal separation at a centrifugal force of 10000 G for 10 minutes to produce solids.
7) Add to the solids produced in step 6) a 60% by mass aqueous solution of ethanol in such an amount that the mass of the alcohol is three times as large as that of the solids.
8) Stir for 5 minutes at 4°C the mixture formed in step 7), and subject the stirred to centrifugal separation at 4°C at a centrifugal force of 10000 G for 10 minutes to produce solids.
9) Subject the solids that have been treated in step 8) again to the operations of steps 7) and 8).

The protein-separating process (A) was completed, as explained in the preceding paragraph. The next process was: 10) Add to the solids obtained in step 9) a 25% by mass aqueous solution of ethanol in such an amount that the mass of the alcohol is three times as large as that of the solids.
11) Stir the mixture produced in step 10) for 5 minutes at 4°C, and subj ect the stirredmixture, being kept at 4°C, to centrifugal separation at a centrifugal force of 10000 G for 10 minutes to produce solids.
12) Subject the solids produced in step 11) to a series of the operations of steps 10) and 11) twice. Divide the treated into a solid constituent and a liquid constituent.
13) Subject the liquid constituent separated in step 12) to dialysis and freeze-drying to produce a final solid product (L) . Place the solid constituent separated in step 12) in water to form a suspension, and subject the suspension to dialysis and freeze-drying to produce a final solid product (S).

The respective yields of the produced mucin and collagen are shown in Table 2.

### (Comparative Example 1)

The same operations as the operations of steps 1) to 4) of Working Example 2 were carried out and a liquid component was produced. Ethanol was added to this liquid component so that the concentration of the ethanol to the resulting mixture was 50% by volume, whereby precipitate in the form of a gel was formed. The liquid containing the gel precipitate was subjected to the same operations as the operations of steps 6) to 13) of Working Example 1. A final solid product (L) and a final solid product (S) were produced.

The respective yields of the produced mucin and collagen are shown in Table 2.

### (Working Example 3)

The same operations as the operations of steps 1) to 4) of Working Example 2 were carried out and a liquid component was produced. Ethanol was added to this liquid component so that the concentration of the ethanol to the resulting mixture was 60% by volume, whereby precipitate in the form of a gel was formed. The liquid containing the gel precipitate was subjected to the same operations as the operations of steps 6) to 13) of Working Example 1. A final solid product (L) and a final solid product (S) were produced.

The respective yields of the produced mucin and collagen are shown in Table 2.

### (Comparative Example 2)

The same procedure as that of Working Example 2 was followed except that step 10) was so changed that isopropanol was added to the solids produced in the preceding step in such an amount that the concentration of the isopropanol was 60% by mass to the mass of the solids. A final solid product (L) and a final solid product (S) were produced.

The respective yields of the produced mucin and collagen are shown in Table 2.

**[Table 2]**

| Yield (%) | Working Example 1 | Working Example 2 | Working Example 3 | Comparative Example 1 | Comparative Example 2 |
|---|---|---|---|---|---|
| Raw material jellyfish (wet weight) | 100 | 100 | 100 | 100 | 100 |
| Mucin | 0.015 | 0.018 | 0.015 | 0.001 | 0.000 |
| Collagen | 0.140 | 0.195 | 0.195 | 0.089 | 0.190 |

The results of Working Examples 1-3 and Comparative Examples 1-2 clearly show that the method of this invention is capable of fractionally extracting mucin and collagen at high efficiency.

### (Comparative Example 3)

The same procedure as that of Working Example 2 was followed except that step 10) was so changed that water was added to the solids produced in the preceding step in such an amount that the mass of the water was three times as large as that of the solids. The liquid constituent obtained in the final step 12) of the production was dialyzed and freeze-dried, so that a final solid product (Lc) was produced. Also, the solid constituent obtained in step 12) was placed in water to form a suspension. The suspension was dialyzed and freeze-dried, so that a final solid product (Sc) was produced. The respective yields of the produced mucin and collagen are shown in Table 3.

An amino acid analysis of the final solid product (Lc) and the final solid product (Sc) was carried out with an automatic amino acid analyzer in accordance with the same procedure as in Working Example 1.

The result of the analysis of composition of the amino acids is shown in Table 4. As understood from the column whose header is "Concentration ratio of amino acids", the analysis revealed that the peptide of the mucin, produced as the solid product (Lc), had such a composition that the ratio of threonine (Thr) : glutamic acid (Glu) : proline (Pro) : alanine (Ala) : valine (Val) +isoleucine (Ile) : N-acetylgalactosamine (GalNAc) was far from 2:1:1:2:2:2. It also showed that the proportion of the concentration of glycine (Gly), which originated in collagen, was high. These results confirm that the produced mucin had low purity.

**[Table 3]**

| Yield (%) | Comparative Example 3 |
|---|---|
| Raw material jellyfish (wet weight) | 100 |
| Mucin | 0.040 |
| Collagen | 0.036 |

**[Table 4]**

| Amino acid associated with peak | Concentration (nmol) | Concentration ratio |
|---|---|---|
| Asp | 1 | |
| Thr | 0.6 | 0.55 |
| Ser | 0.6 | |
| Glu | 1.1 | 1.1 |
| Pro | 1 | 1 |
| Gly | 3.6 | 3.59 |
| Ala | 1.1 | 1.14 |
| Cys | 0 | |
| Val | 0.5 | 0.49 |
| Met | 0.1 | |
| Ile | 0.3 | |
| Leu | 0.4 | |
| Tyr | 0.1 | |
| Phe | 0.1 | |
| Lys | 0.5 | |
| His | 0 | |
| Arg | 0.6 | |
| Total | 11.6 | |
| GlcNH2 | 0.1 | |
| GalNH2 | 0.1 | |

### (Referential Example)

Ethanol was added to the liquid component produced in step 4) of Working Example 1 so that the concentration of the ethanol to the resultant was 20% by volume, and the resultant was mixed. Precipitate was formed. The liquid containing the precipitate was subjected to solid-liquid separation and it was divided into the precipitate and a liquid portion, which will be called first liquid portion hereinafter. An amino acid analysis of the precipitate was carried out with an automatic amino acid analyzer in accordance with the same procedure as in Working Example 1. As a result, the analysis revealed that the main component shown in Table 5 was produced in the yield also shown in Table 5.

Ethanol was added to the first liquid portion so that the concentration of the ethanol to the resultant was 30% by volume, and the resultant was mixed. Precipitate was formed. The liquid containing the precipitate was subjected to solid-liquid separation and it was divided into the precipitate and a liquid portion, which will be called second liquid portion hereinafter. An amino acid analysis of the precipitate was carried out with an automatic amino acid analyzer in accordance with the same procedure as in Working Example 1. As a result, the analysis revealed that the main component shown in Table 5 was produced in the yield also shown in Table 5.

Ethanol was added to the second liquid portion so that the concentration of the ethanol to the resultant was 40% by volume, and the resultant was mixed. Precipitate was formed. The liquid containing the precipitate was subjected to solid-liquid separation and it was divided into the precipitate and a liquid portion, which will be called third liquid portion hereinafter. An amino acid analysis of the precipitate was carried out with an automatic amino acid analyzer in accordance with the same procedure as in Working Example 1. As a result, the analysis revealed that the main component shown in Table 5 was produced in the yield also shown in Table 5.

Ethanol was added to the third liquid portion so that the concentration of the ethanol to the resultant was 50% by volume, and the resultant was mixed. Precipitate was formed. The liquid containing the precipitate was subjected to solid-liquid separation and it was divided into the precipitate and a liquid portion, which will be called fourth liquid portion hereinafter. An amino acid analysis of the precipitate was carried out with an automatic amino acid analyzer in accordance with the same procedure as in Working Example 1. As a result, the analysis revealed that the main component shown in Table 5 was produced in the yield also shown in Table 5.

Ethanol was added to the fourth liquid portion so that the concentration of the ethanol to the resultant was 60% by volume, and the resultant was mixed. Precipitate was formed. The liquid containing the precipitate was subjected to solid-liquid separation and it was divided into the precipitate and a liquid portion, which will be called fifth liquid portion hereinafter. An amino acid analysis of the precipitate was carried out with an automatic amino acid analyzer in accordance with the same procedure as in Working Example 1. As a result, the analysis revealed that the main component shown in Table 5 was produced in the yield also shown in Table 5.

Ethanol was added to the fifth liquid portion so that the concentration of the ethanol to the resultant was 70% by volume, and the resultant was mixed. Precipitate was formed. The liquid containing the precipitate was subjected to solid-liquid separation and it was divided into the precipitate and a liquid portion, which will be called fifth liquid portion hereinafter. An amino acid analysis of the precipitate was carried out with an automatic amino acid analyzer in accordance with the same procedure as in Working Example 1. As a result, the analysis revealed that the main component shown in Table 5 was produced in the yield also shown in Table 5.

Another series of the same experiments were also carried out except that propanol was used in place of ethanol. The results had the same tendency as that shown in Table 5.

**[Table 5]**

| Concentration of ethanol (vol%) | Yield (mg) | Main component |
|---|---|---|
| 20 | 3108.1 | Collagen |
| 30 | 308.5 | Collagen |
| 40 | 518.1 | Several kinds of peptides |
| 50 | 2918.0 | Mucin |
| 60 | 190.4 | Mucin |
| 70 | 55.2 | Mucin |

### INDUSTRIAL APPLICABILITY

The present invention provides a method for fractionally extracting mucin and collagen from jellyfish at high efficiency. Mucin and collagen produced by the method of the invention may be useful in the fields of pharmaceuticals, cosmetics, food products, and agriculture. Mucin and collagen are also capable of being used as raw material for reagents and products thereof.

## Claims

1. A method for fractionally extracting mucin and collagen including:
(A) a protein-separating process comprising breaking a jellyfish to provide broken pieces containing water; subj ecting the broken pieces to first solid-liquid separation to provide a liquid component; mixing the liquid component with a water-soluble alcohol having 1 to 4 carbon atoms to prepare a first mixture wherein the water-soluble alcohol has a concentration of at least 50% by volume to a whole of the first mixture; and subjecting the first mixture to second solid-liquid separation to provide solids, and
(B) a fractionating process comprising mixing the solids, which have been provided in the protein-separating process (A), with an aqueous solution of a water-soluble alcohol having 1 to 4 carbon atoms wherein a concentration of the alcohol in the aqueous solution is from 10 to 40% by mass, to provide a second mixture; and subjecting the secondmixture to third solid-liquid separation whereby the second mixture is fractionally divided into a mucin-containing liquid constituent and a collagen-containing solid constituent.

2. The method for fractionally extracting mucin and collagen according to claim 1, the protein-separating process (A) further comprising washing the solids provided by the second solid-liquid separation with a water-soluble alcohol having 1-4 carbon atoms and/or water to provide washed solids, which are supplied to the fractionating process (B).

3. The method for fractionally extracting mucin and collagen according to claim 2, wherein the water-soluble alcohol having 1-4 carbon atoms and/or water is so mixed with the washed solids produced in the protein-separating process (A) that a mass of the water-soluble alcohol having 1-4 carbon atoms and/or water is 1 to 4 times a mass of the washed solids.

## Patentansprüche

1. Verfahren zur fraktionierten Extraktion von Mucin und Kollagen, beinhaltend:
(A) ein Protein-Trennverfahren aufweisend das Brechen einer Qualle, um Bruchstücke bereitzustellen, welche Wasser enthalten; Unterziehen der Bruchstücke einer ersten Fest-Flüssig-Trennung, um eine flüssige Komponente bereitzustellen; Mischen der flüssigen Komponente mit einem wasserlöslichen Alkohol, welcher 1 bis 4 Kohlenstoffatome hat, um eine erste Mischung herzustellen, wobei der wasserlösliche Alkohol eine Konzentration von mindestens 50 Volumenprozent bezüglich einer Gesamtheit der ersten Mischung hat; und Unterziehen der ersten Mischung einer zweiten Fest-Flüssig-Trennung, und
(B) ein Fraktionierungsverfahren aufweisend das Mischen der Feststoffe, welche im Protein-Trennverfahren (A) bereitgestellt wurden, mit einer wässrigen Lösung eines wasserlöslichen Alkohols, welcher 1 bis 4 Kohlenstoffatome hat, wobei eine Konzentration des Alkohols in der wässrigen Lösung von 10 bis 40 Massenprozent ist, um eine zweite Mischung bereitzustellen; und Unterziehen der zweiten Mischung einer dritten Fest-Flüssig-Trennung, wodurch die zweite Mischung fraktionell in einen Mucin-enthaltenden flüssigen Bestandteil und einen Kollagen-enthaltenden festen Bestandteil getrennt wird.

2. Verfahren zur fraktionierten Extraktion von Mucin und Kollagen gemäss Anspruch 1, wobei das Protein-Trennverfahren (A) weiter aufweist das Waschen der Feststoffe, welche durch die zweite Fest-Flüssig-Trennung bereitgestellt werden, mit einem wasserlöslichen Alkohol, welcher 1-4 Kohlenstoffatome hat, und/oder Wasser, um gewaschene Feststoffe bereitzustellen, welche dem Fraktionierungsverfahren (B) zugegeben werden.

3. Verfahren zur fraktionierten Extraktion von Mucin und Kollagen gemäss Anspruch 2, wobei der wasserlösliche Alkohol, welcher 1-4 Kohlenstoffatome hat, und/oder Wasser derart mit den gewaschenen Feststoffen, welche im Protein-Trennverfahren (A) hergestellt werden, gemischt werden, dass eine Masse des wasserlöslichen Alkohols, welcher 1-4 Kohlenstoffatome hat, und/oder Wasser 1 bis 4 Mal eine Masse der gewaschenen Feststoffe ist.

## Revendications

1. Un procédé pour l'extraction fractionnaire de mucine et de collagène, incluant :
(A) un processus de séparation de protéines comprenant écraser des méduses pour fournir des pièces écrasées contenant de l'eau ; soumettre les pièces écrasées à une première séparation solide-liquide pour fournir un composant liquide ; mélanger le composant liquide avec un alcool hydrosoluble ayant de 1 à 4 atomes de carbone pour préparer un premier mélange, dans lequel l'alcool hydrosoluble a une concentration d'au moins 50% en volume par rapport à l'ensemble du premier mélange, et soumettre le premier mélange à une deuxième séparation solide-liquide pour fournir des solides, et
(B) un processus de fractionnement comprenant mélanger les solides, qui ont été fournis dans le processus de séparation de protéines (A), avec une solution aqueuse d'un alcool hydrosoluble ayant de 1 à 4 atomes de carbone, dans lequel une concentration de l'alcool dans la solution aqueuse est de 10 % à 40 % en masse, pour fournir un deuxième mélange ; et soumettre le deuxième mélange à une troisième séparation solide-liquide selon lequel le deuxième mélange est fractionnellement divisé en un constituant liquide contenant de la mucine et un constituant solide contenant du collagène.

2. Le procédé pour l'extraction fractionnaire de mucine et de collagène selon la revendication 1, le processus de séparation de protéines (A) comprenant d'avantage laver les solides fournis par la deuxième séparation solide-liquide avec un alcool hydrosoluble ayant de 1 à 4 atomes de carbone et/ou de l'eau pour fournir des solides lavés, qui sont amené au processus de fractionnement (B).

3. Le procédé pour l'extraction fractionnaire de mucine et de collagène selon la revendication 2, dans lequel l'alcool hydrosoluble ayant de 1 à 4 atomes de carbone et/ou l'eau est mélangé(e) avec les solides lavés produits dans le processus de séparation de protéine (A) de sorte que la masse de l'alcool hydrosoluble ayant de 1 à 4 atomes de carbone et/ou de l'eau est de 1 à 4 fois la masse des solides lavés.
